# EUROPEAN PATENT APPLICATION

(11) **EP 4 212 193 A1**
(43) Date of publication of application: **19.07.2023**
(21) Application number: 22206807.4
(22) Date of filing: 10.11.2022
(51) Int. Cl.: A61M 5/00, B65D 85/00

(54) **MEDICAL DEVICE PACKAGE**

(30) Priority: 18.01.2022 JP 2022005540
(71) Applicant: Terumo Kabushiki Kaisha, Tokyo, 1510072 (JP)
(72) Inventor: HASUMI, Kiyoaki, Showa-cho, 4093853 (JP)
(74) Representative: Casalonga

(57) **Abstract**

A medical device package includes a container main body and a sealing film that covers and seals a recess portion of the container main body, and the medical device is accommodated in the recess portion of the container main body. The sealing film includes an unsealing assistance portion for facilitating breakage of a part of the sealing film. At least a part of the unsealing assistance portion faces a take-out recess portion of the container main body.

## Description

### BACKGROUND

### Technical Field

The present invention relates to a medical device package that accommodates a medical device inside a packaging container.

### Related Art

There is known a medical device package that stores a medical device in a recess portion formed on a resin sheet. The packaging container in JP 2011-005182 A includes a main body having a recess portion and a sheet-like lid covering an opening portion of the main body, and a medical device such as a liquid medicine administration device or a prefilled syringe is accommodated in the recess portion and sealed by the lid. After peeling off the lid from the main body to open the recess portion, an unsealing person takes out the medical device from the recess portion and uses the medical device.

### SUMMARY

In the packaging container of JP 2011-005182 A, when the medical device is taken out from the recess portion of the main body, it is necessary to peel off the lid from the main body, and the unsealing person needs to grasp the main body with one hand and peel off the lid with the other hand. Therefore, the operation at the time of taking out the medical device from the packaging container is complicated. Since the entire recess portion is opened when the lid is peeled off from the main body, there is a concern that the medical device may fall from the opened recess portion.

An object of the present invention is to solve the above-described problem.

According to an aspect of the present invention, there is provided a medical device package including a medical device and a packaging container that accommodates the medical device,
in which the packaging container includes
a bottomed container main body including an accommodating recess portion accommodating the medical device and an opening end portion surrounding the accommodating recess portion, and
a sealing film formed in a sheet shape and bonded to the opening end portion of the container main body to seal the accommodating recess portion,
the container main body includes a take-out recess portion provided to be adjacent to the medical device and the accommodating recess portion and capable of receiving a finger of an unsealing person, and
the sealing film includes an unsealing assistance portion for facilitating breakage of a part of the sealing film at least at a position facing the take-out recess portion.

According to the aspect of the present invention, the following effects can be obtained.

That is, the container main body of the medical device package includes the take-out recess portion that is adjacent to the medical device and the accommodating recess portion and capable of receiving the finger of the unsealing person, and the sealing film includes the unsealing assistance portion at a position facing the take-out recess portion. Therefore, since the sealing film is broken by the finger of the unsealing person through the unsealing assistance portion, the finger is inserted into the take-out recess portion to move the medical device toward the opening end portion, and then the medical device can be taken out to the outside from the opening portion of the broken sealing film.

As a result, the medical device can be taken out to the outside without peeling off the sealing film from the sticking portion of the container main body. Only a part of the sealing film is opened by using the unsealing assistance portion, and thus it is possible to prevent the medical device from falling through the opening portion.

### BRIEF DESCRIPTION OF DRAWINGS

FIG. 1 is an external perspective view of a medical device package according to an embodiment of the present invention;
FIG. 2 is an exploded perspective view of the medical device package illustrated in FIG. 1;
FIG. 3 is a plan view of the medical device package illustrated in FIG. 1;
FIG. 4 is a cross-sectional view of the medical device package illustrated in FIG. 3;
FIG. 5 is an operation explanatory view illustrating a state before a medical device is taken out from a medical device package;
FIG. 6 is an operation explanatory view illustrating a state in which a thumb of an unsealing person breaks a part of a sealing film and the thumb is inserted;
FIG. 7 is an operation explanatory view illustrating a state in which a base end of a medical device is held by a thumb in the medical device package of FIG. 6;
FIG. 8 is an operation explanatory view illustrating a state in which a medical device is taken out from a medical device package;
FIG. 9 is an overall plan view of a medical device package according to a first modification example; and
FIG. 10 is an overall plan view of a medical device package according to a second modification example.

### DETAILED DESCRIPTION

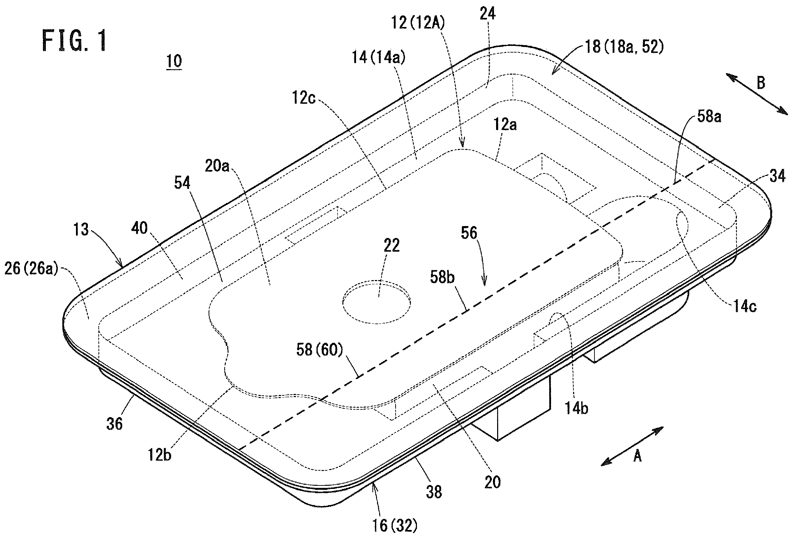

As illustrated in FIGS. 1 to 4, a medical device package 10 includes a medical device 12 and a packaging container 13. In the present embodiment, the medical device 12 is a small patch-type liquid medicine administration device 12A that incorporates a container accommodating a liquid medicine and a pump for pushing out the liquid medicine, and is used by being attached to the body surface of a patient. The medical device 12 accommodated in the packaging container 13 is not limited to the liquid medicine administration device 12A, and examples of the liquid medicine administration device include a wearable-type liquid medicine administration device that incorporates a container accommodating a liquid medicine and a pump for pushing out the liquid medicine and is installed to clothing to be used, an auto-injector, a prefilled syringe, an indwelling needle, and a catheter.

The liquid medicine administration device 12A includes a box-shaped casing 20. A cylinder (not illustrated) filled with a liquid medicine is accommodated in the casing 20. An upper surface 20a of the casing 20 includes a power switch 22. The upper surface 20a of the casing 20 is a surface to be attached to the body surface of the patient. When the unsealing person presses the power switch 22, the liquid medicine in the cylinder is continuously or intermittently administered into a living body for a relatively long time (for example, about several minutes to several hours).

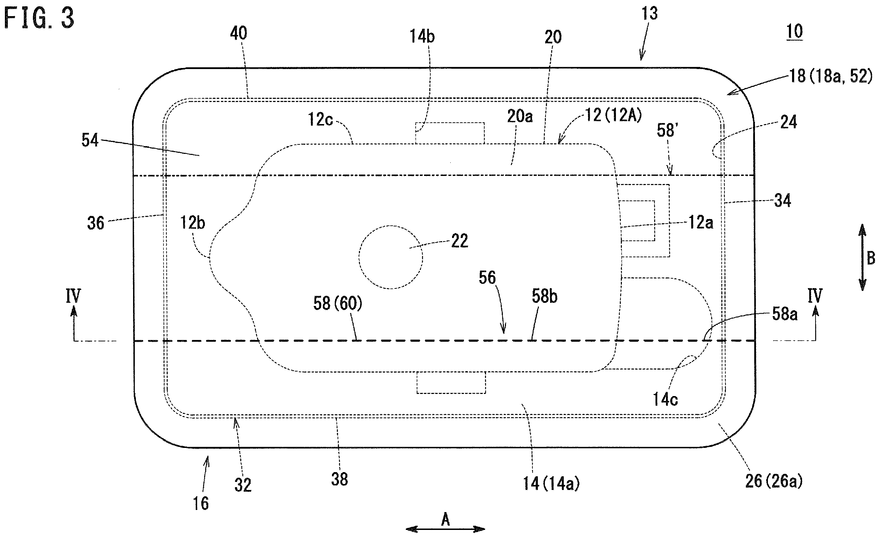

The packaging container 13 includes a container main body 16 having a recess portion 14 and a sealing film 18. The container main body 16 is formed in a rectangular shape in a plan view (see FIG. 3). Hereinafter, a long length direction of the container main body 16 is referred to as a longitudinal direction (direction of arrow A). A short length direction of the container main body 16 orthogonal to the longitudinal direction is referred to as a width direction (direction of arrow B).

The container main body 16 includes a recess portion 14 and an opening end portion 24. The opening end portion 24 is disposed at an upper end of the container main body 16 and is formed in a rectangular shape along an outer peripheral portion of the container main body 16. The opening end portion 24 has a sticking portion 26. The sticking portion 26 has a flange shape extending in a direction away from the recess portion 14 at the opening end portion 24. The sticking portion 26 has a sticking surface 26a parallel to a bottom portion 28 of the recess portion 14. An outer edge portion 52 of the sealing film 18 to be described later is fused to the sticking surface 26a of the sticking portion 26.

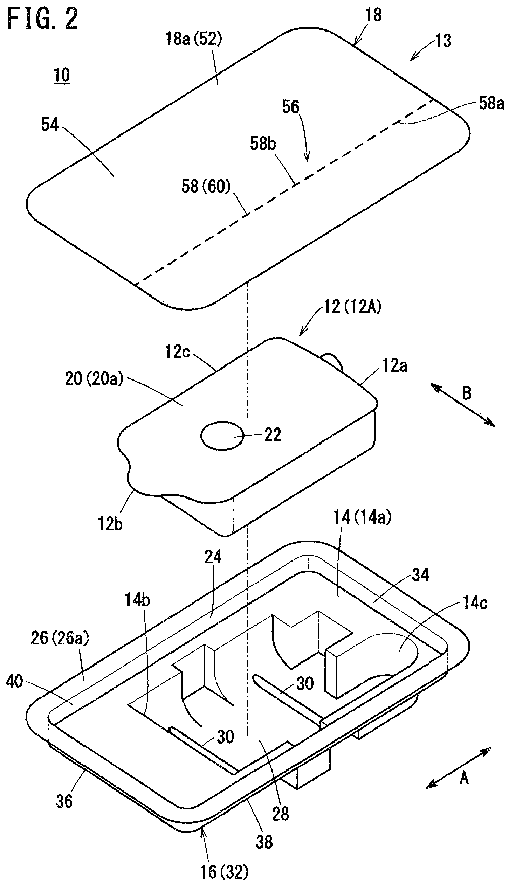

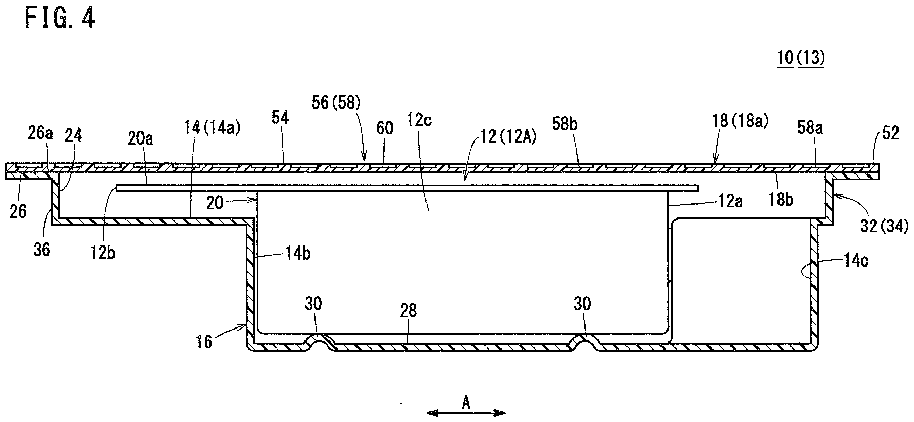

The recess portion 14 is recessed downward from the opening end portion 24 (see FIGS. 2 and 4). The recess portion 14 includes a recess portion main body 14a, an accommodating recess portion 14b, and a take-out recess portion 14c. The recess portion main body 14a has a rectangular shape in a plan view. The recess portion main body 14a has a flat bottom surface recessed from the opening end portion 24 at a constant depth.

The accommodating recess portion 14b is recessed downward from the bottom surface of the recess portion main body 14a. In a plan view of the container main body 16, the accommodating recess portion 14b is formed in a shape corresponding to the outer shape of the casing 20. A lower portion of the casing 20 is accommodated inside the accommodating recess portion 14b. An upper portion of the casing 20 is accommodated in the recess portion main body 14a. When the medical device 12 is accommodated in the container main body 16, the lower portion of the casing 20 is accommodated in the accommodating recess portion 14b, and thus the casing 20 is held at a predetermined position without moving in the recess portion 14.

A plurality of protrusions 30 are disposed on the bottom portion 28 of the accommodating recess portion 14b. Each of the protrusions 30 protrudes from the bottom portion 28 of the accommodating recess portion 14b toward the opening end portion 24. When the medical device 12 is accommodated in the accommodating recess portion 14b of the recess portion 14, the bottom surface of the casing 20 is held by the protrusion 30. A distal end 12b and a base end 12a of the medical device 12 are disposed in the longitudinal direction of the container main body 16.

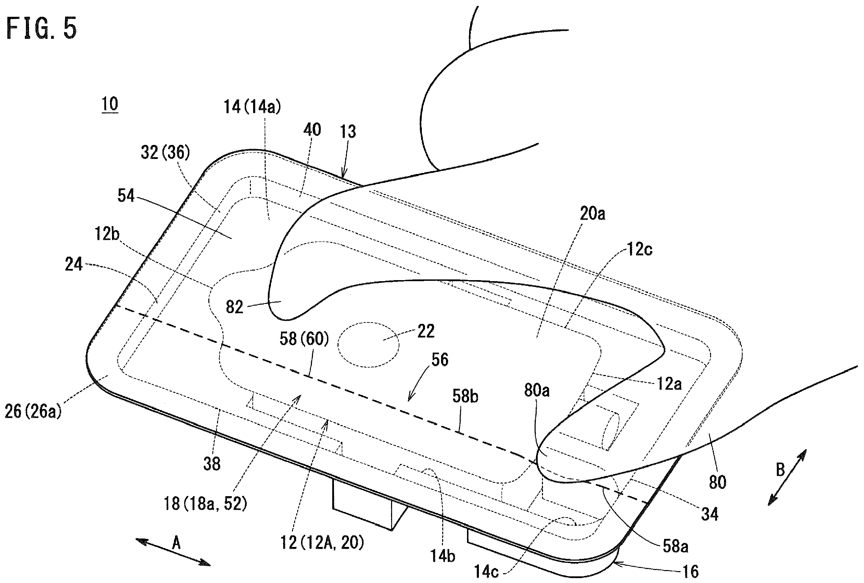

The take-out recess portion 14c is disposed adjacent to the accommodating recess portion 14b. In order to break the sealing film 18 and take out the medical device 12 from the recess portion 14, the take-out recess portion 14c can receive a thumb 80 of the unsealing person (see FIG. 5). The take-out recess portion 14c is disposed closer to one side in the width direction from a center of the accommodating recess portion 14b in the width direction (see FIG. 3). The take-out recess portion 14c is recessed downward from the recess portion main body 14a. The take-out recess portion 14c and the accommodating recess portion 14b have substantially the same depth. The take-out recess portion 14c and the base end of the accommodating recess portion 14b communicate with each other in the longitudinal direction of the container main body 16.

The recess portion 14 has a side wall 32 orthogonal to the bottom surface of the recess portion main body 14a. The side wall 32 connects the opening end portion 24 with the bottom surface of the recess portion main body 14a.

The side wall 32 includes a base end wall portion 34, a distal end wall portion 36, a first width direction wall portion 38, and a second width direction wall portion 40. That is, the side wall 32 is formed of four wall portions of the base end wall portion 34, the distal end wall portion 36, and the first and second width direction wall portions 38 and 40.

The base end wall portion 34 is disposed at the base end of the container main body 16 (recess portion 14). The base end wall portion 34 extends in the width direction (direction of arrow B) of the container main body 16 and faces the base end 12a of the medical device 12. The distal end wall portion 36 is disposed at the distal end of the container main body 16 (recess portion 14). The distal end wall portion 36 extends in the width direction of the container main body 16 and faces the distal end 12b of the medical device 12.

The first width direction wall portion 38 is disposed at one end of the container main body 16 (recess portion 14) in the width direction. The second width direction wall portion 40 is disposed at the other end of the container main body 16 (recess portion 14) in the width direction. Each of the first and second width direction wall portions 38 and 40 extends in the longitudinal direction (direction of arrow A) of the container main body 16.

The sealing film 18 is formed of a resin material such as polyethylene resin. The sealing film 18 has a thin sheet shape having flexibility. In a plan view of the medical device package 10 illustrated in FIG. 3, the sealing film 18 has a rectangular shape having substantially the same dimension as that of the container main body 16.

The sealing film 18 has an outer edge portion 52, a covering portion 54, and an unsealing assistance portion 56. The outer edge portion 52 has an annular shape along the outer edge of the sealing film 18. The sealing film 18 is disposed so as to cover the opening end portion 24 and the recess portion 14 of the container main body 16, and the outer edge portion 52 of the sealing film 18 is superposed on the sticking portion 26 and heated. Accordingly, the outer edge portion 52 of the sealing film 18 and the sticking surface 26a of the container main body 16 are bonded (fused). The medical device 12 is sealed inside the container main body 16 (recess portion 14) by the container main body 16 and the sealing film 18 (see FIG. 4).

The covering portion 54 of the sealing film 18 is surrounded by the outer edge portion 52. When the sealing film 18 is fused to the container main body 16, the covering portion 54 faces the recess portion 14. At this time, the covering portion 54 and the container main body 16 are not in contact with each other, and the covering portion 54 faces the medical device 12 (recess portion 14).

The unsealing assistance portion 56 is provided on the covering portion 54. When the unsealing person (not illustrated) takes out the medical device 12 from the container main body 16, the sealing film 18 is easily broken through the unsealing assistance portion 56 and the container main body 16 is unsealed.

The unsealing assistance portion 56 has a breaking line 58 for cutting the sealing film 18 in a thickness direction. The breaking line 58 extends in the longitudinal direction (direction of arrow A) of the sealing film 18. The breaking line 58 is disposed closer to one side of the sealing film 18 in the width direction from the center of the sealing film 18 in the width direction. The breaking line 58 is linear in the longitudinal direction (direction of arrow A) of the sealing film 18. The breaking line 58 faces the take-out recess portion 14c of the recess portion 14.

The breaking line 58 is formed of perforations having a plurality of cut-outs 60 obtained by cutting out the sealing film 18. A plurality of the cut-outs 60 are intermittently disposed at predetermined intervals in the longitudinal direction (direction of arrow A) of the sealing film 18. As illustrated in FIG. 4, the cut-outs 60 of the breaking line 58 are disposed on a front surface 18a of the sealing film 18 opposite to a back surface 18b of the sealing film 18 to be fused. The breaking line 58 may be formed of perforations having a penetrating portion penetrating in the thickness direction of the sealing film 18. By breaking the breaking line 58, a part of the sealing film 18 (covering portion 54) can be broken along the breaking line 58 in the longitudinal direction.

The breaking line 58 includes a breaking start portion 58a and a breaking extension portion 58b. The breaking start portion 58a is disposed at the base end of the breaking line 58 and is disposed inside the outer edge portion 52 of the container main body 16. In a plan view of the medical device package 10 illustrated in FIG. 3, the breaking start portion 58a faces the take-out recess portion 14c. When the medical device 12 is taken out from the container main body 16, the breaking start portion 58a is pressed by the thumb 80 of the unsealing person.

The breaking extension portion 58b is connected to and continuous with the distal end of the breaking start portion 58a. The breaking extension portion 58b extends from the distal end of the breaking start portion 58a toward the base end of the breaking start portion 58a. The breaking extension portion 58b extends to an inner side of the outer edge portion 52 of the container main body 16 at the base end of the breaking line 58. The number of breaking lines 58 is not limited to one, and may be plural. In the plan view illustrated in FIG. 3, two breaking lines 58 (58') may be disposed apart from each other in the width direction (direction of arrow B) with the medical device 12 interposed therebetween.

The breaking line 58 may have only the breaking start portion 58a (breaking extension portion 58b may not be provided). In particular, in a case where the sealing film 18 is a uniaxially stretched film stretched in one direction (for example, direction of arrow A), the sealing film 18 is easily torn in the one direction. Therefore, even in a case where the breaking extension portion 58b is not provided, when the breaking start portion 58a is broken, the breaking length of the sealing film 18 can be further increased starting from the breaking start portion 58a.

Next, a case of taking out the medical device 12 (liquid medicine administration device 12A) from the medical device package 10 will be described with reference to FIGS. 5 to 8.

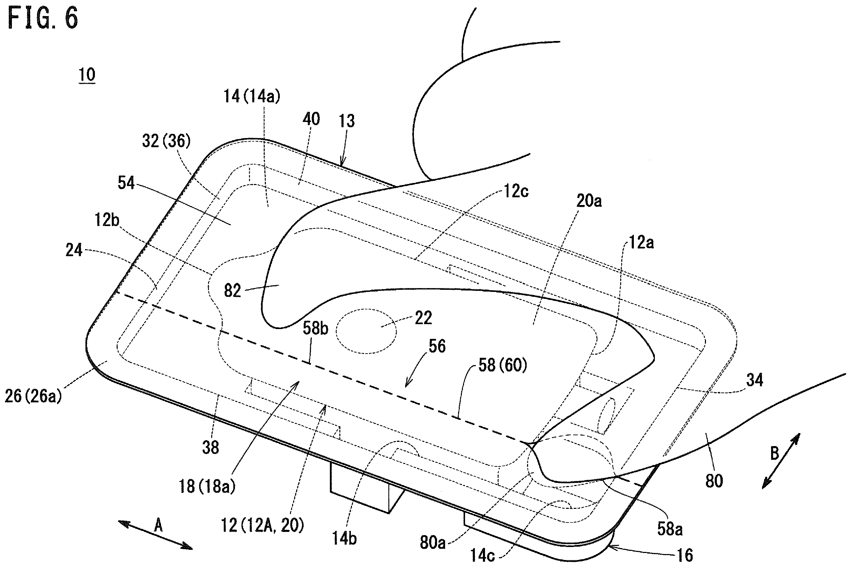

As illustrated in FIG. 5, after the medical device package 10 is placed with the sealing film 18 as an upper side, a tip 80a of the thumb 80 of the unsealing person is brought into contact with the breaking start portion 58a of the breaking line 58. As illustrated in FIG. 6, by being pressed by the tip 80a of the thumb 80 toward the inside of the container main body 16, the breaking start portion 58a of the breaking line 58 is broken by the tip 80a of the thumb 80.

The tip 80a of the thumb 80 is inserted into the take-out recess portion 14c of the container main body 16 through the broken breaking start portion 58a. The tip 80a of the thumb 80 faces the base end 12a of the medical device 12 in the take-out recess portion 14c. At this time, an index finger 82 of the unsealing person is brought into contact with a portion in the vicinity of the breaking extension portion 58b on the front surface 18a of the sealing film 18. The distal end 12b of the medical device 12 is held by the index finger 82 via the sealing film 18.

The sealing film 18 is torn and bifurcates in the width direction (direction of arrow B) along the breaking extension portion 58b from the breaking start portion 58a. In the vicinity of the breaking start portion 58a, the sealing film 18 is separated into a first breaking portion 62 separated toward one side of the width direction and a second breaking portion 64 separated toward the other side of the width direction. The first breaking portion 62 and the second breaking portion 64 are pressed and expanded to opposite sides in the width direction by the thumb 80, and thus the first breaking portion 62 and the second breaking portion 64 are separated from each other. An opening portion 66 opened between the first breaking portion 62 and the second breaking portion 64 is formed on the sealing film 18. A portion in the vicinity of the base end 12a of the medical device 12 is exposed to the outside through the opening portion 66.

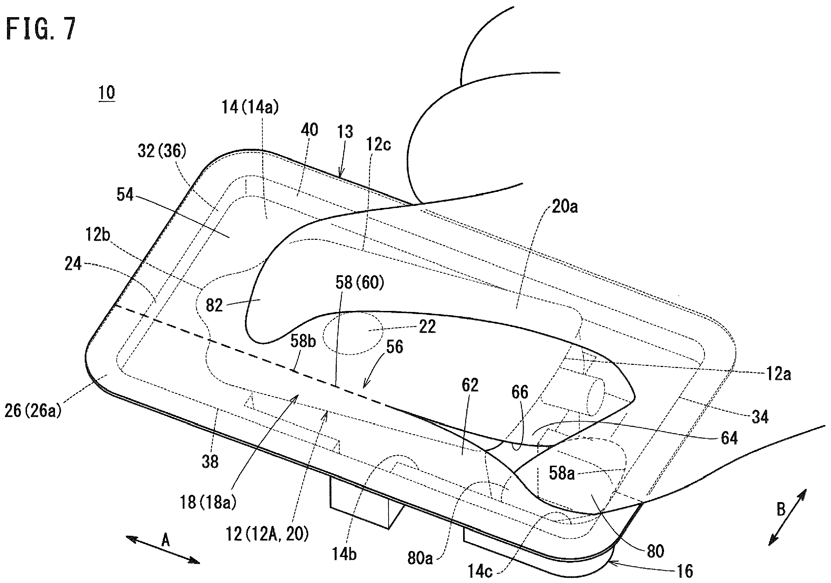

In the take-out recess portion 14c, the tip 80a of the thumb 80 is inserted into the lower portion of the medical device 12. As illustrated in FIG. 7, the upper surface of the distal end 12b of the medical device 12 is pressed by the index finger 82, and the base end 12a of the medical device 12 is lifted upward (toward the sealing film 18) in a belly portion (inner side) of the thumb 80. Accordingly, the medical device 12 is inclined such that the base end 12a faces upward, and the base end 12a is taken out upward through the opening portion 66 between the first breaking portion 62 and the second breaking portion 64. The distal end 12b of the medical device 12 is still accommodated in the recess portion 14.

As illustrated in FIG. 7, when the base end 12a of the medical device 12 is taken out upward from the opening portion 66, the sealing film 18 is continuously broken from the breaking start portion 58a along the breaking extension portion 58b by an intermediate portion 12c and the base end 12a of the medical device 12. When the breaking line 58 is broken as the medical device 12 is taken out upward, the first and second breaking portions 62 and 64 are gradually expanded toward the distal end of the breaking extension portion 58b. As the first and second breaking portions 62 and 64 are expanded, the opening area of the opening portion 66 gradually increases toward the distal end. That is, as the base end 12a of the medical device 12 is taken out from the container main body 16, the sealing film 18 is gradually broken toward the distal end by the medical device 12.

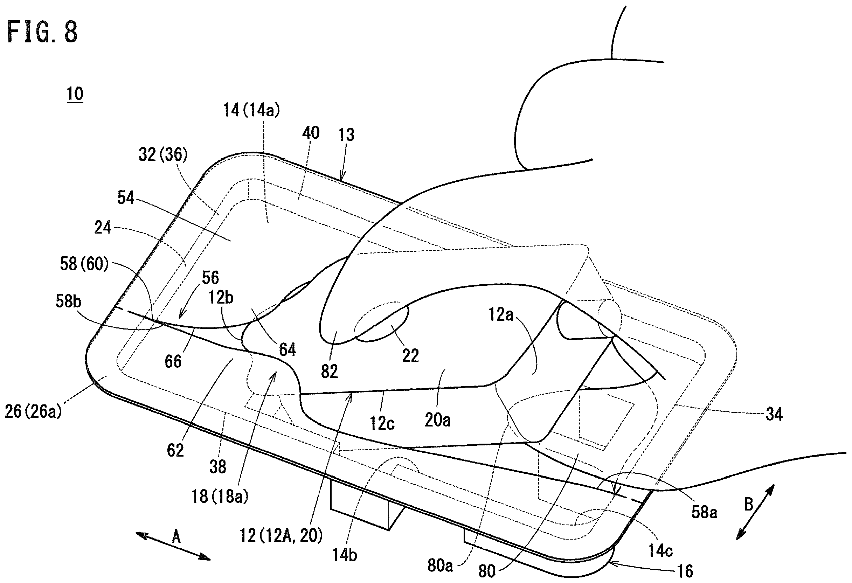

The medical device 12 is taken out, the breaking line 58 is broken to a portion in the vicinity of the distal end of the breaking extension portion 58b, and the opening portion 66 is expanded to the vicinity of the distal end of the sealing film 18, after that, as illustrated in FIG. 8, the unsealing person grasps the base end 12a of the medical device 12 with the thumb 80 and the index finger 82 and takes out the medical device 12 upward. Specifically, the thumb 80 holds the lower portion of the medical device 12 in the accommodating recess portion 14b, the index finger 82 holds the upper portion of the medical device 12, and the thumb 80 and the index finger 82 vertically pinches the casing 20 of the medical device 12.

When the unsealing person takes out the medical device 12 upward, the medical device 12 is taken out to the outside of the container main body 16 from the opening portion 66 of the sealing film 18 of which the breaking line 58 is broken and opened in the longitudinal direction.

At this time, the first breaking portion 62 of the sealing film 18 is fused to the sticking portion 26 of the container main body 16 on one side of the breaking line 58 in the width direction, and the second breaking portion 64 is fused to the sticking portion 26 of the container main body 16 on the other side of the breaking line 58 in the width direction. That is, in the sealing film 18, only a part of the covering portion 54 having the unsealing assistance portion 56 is broken, and the opening portion 66 is opened.

Therefore, the sealing film 18 is not opened at a portion other than the opening portion 66, and the medical device 12 is taken out to the outside only through the opening portion 66.

As described above, in the embodiment of the present invention, the packaging container 13 includes the container main body 16 having the recess portion 14 and the sealing film 18 covering the opening end portion 24 of the container main body 16, the medical device 12 is accommodated in the accommodating recess portion 14b, and the sealing film 18 includes the unsealing assistance portion 56 for facilitating breakage of a part of the sealing film 18.

In a plan view of the medical device package 10 illustrated in FIG. 3, the container main body 16 includes the take-out recess portion 14c that is adjacent to the medical device 12 and the accommodating recess portion 14b and can receive the thumb 80 of the unsealing person.

Accordingly, since the sealing film 18 is broken by the thumb 80 of the unsealing person through the unsealing assistance portion 56, a part of the sealing film 18 can be unsealed without peeling off the sealing film 18 from the sticking portion 26 of the container main body 16. Therefore, it is not necessary to peel off the sealing film 18 from the container main body 16, and the medical device 12 can be easily taken out to the outside through the opening portion 66 of the sealing film 18 opened by the unsealing assistance portion 56.

When the medical device 12 is taken out from the recess portion 14 of the container main body 16, since only a part of the sealing film 18 is opened by using the unsealing assistance portion 56, the medical device 12 accommodated in the accommodating recess portion 14b of the container main body 16 is prevented from erroneously falling through the opening portion 66 as compared with a packaging container in the related art, of which the entire main body as a container is opened.

Since the unsealing assistance portion 56 has the breaking line 58 cut out in the thickness direction of the sealing film 18 and the breaking line 58 extends along the sealing film 18, the unsealing person can easily and reliably break the sealing film 18 through the breaking line 58 and open the container main body 16 by bring the tip 80a of the thumb 80 into contact with the breaking start portion 58a of the breaking line 58 and pressing the breaking start portion 58a.

When the sealing film 18 is broken by the thumb 80 of the unsealing person via the breaking line 58, the tip 80a of the thumb 80 is inserted into the take-out recess portion 14c of the container main body 16, and thus the base end 12a of the medical device 12 can be made face the thumb 80. Therefore, after the sealing film 18 is broken, the base end 12a of the medical device 12 can be reliably held and taken out upward by the thumb 80.

Since a plurality of the breaking lines 58 and 58' are disposed on the sealing film 18, the sealing film 18 can be more easily and reliably broken by a plurality of the breaking lines 58, and a plurality of the opening portions 66 can be formed in the sealing film 18. Therefore, a large opening area of the sealing film 18 can be secured by a plurality of the opening portions 66, and the medical device 12 can be taken out to the outside more easily through the opening portions 66.

Since the breaking line 58 is formed with perforations and the thumb 80 and the index finger 82 are brought into contact with the breaking line 58 to press, the sealing film 18 can be easily and reliably broken through the breaking line 58 and the opening portion 66 can be opened.

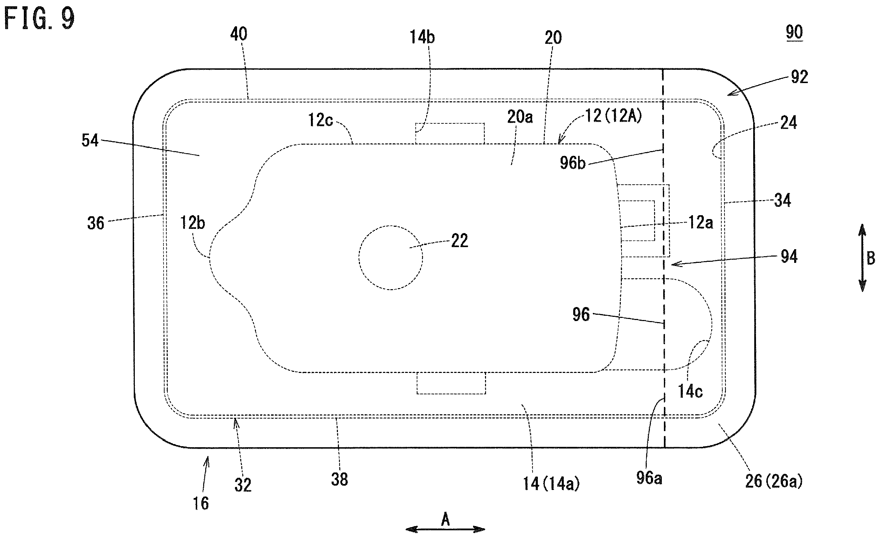

For example, a packaging container 90 according to a first modification example illustrated in FIG. 9 may be employed. In the packaging container 90, the sealing film 92 includes an unsealing assistance portion 94. The unsealing assistance portion 94 has a breaking line 96 extending in the width direction (direction of arrow B) of the sealing film 92. At least a part of the breaking line 96 faces the take-out recess portion 14c of the container main body 16. The breaking line 96 of the sealing film 92 is formed from the first width direction wall portion 38 to the second width direction wall portion 40. The breaking line 96 includes a breaking start portion 96a and a breaking extension portion 96b. In a plan view of the packaging container 90 illustrated in FIG. 9, the breaking start portion 96a faces the take-out recess portion 14c of the container main body 16. The breaking start portion 96a is disposed at one end of the breaking line 96 in the width direction.

When the medical device 12 is taken out from the container main body 16, the tip 80a of the thumb 80 is brought into contact with the breaking start portion 96a of the breaking line 96 and pressing is performed toward the take-out recess portion 14c. Accordingly, the breaking start portion 96a of the breaking line 96 is broken by the tip 80a of the thumb 80, and the tip 80a of the thumb 80 is inserted into the take-out recess portion 14c.

Thereafter, the base end 12a of the medical device 12 is lifted upward by the thumb 80, and thus a part of the base end 12a is taken out to the outside from an opening portion (not illustrated) formed when the breaking start portion 96a is broken. When the medical device 12 is further lifted upward by the thumb 80 of the unsealing person, the sealing film 92 is broken from the breaking start portion 96a toward the breaking extension portion 96b by the medical device 12. When the breaking line 96 is broken toward the distal end, the opening portion of the sealing film 92 gradually expands.

The index finger 82 of the unsealing person is inserted into the recess portion 14 of the container main body 16 through the opening portion, and the base end 12a of the medical device 12 is grasped in the width direction by the thumb 80 and the index finger 82 and taken out from the container main body 16 to the outside.

As described above, in a plan view of the packaging container 90 illustrated in FIG. 9, the sealing film 92 includes the breaking line 96 extending in the width direction (direction of arrow B), and the breaking start portion 96a of the breaking line 96 is disposed to face the take-out recess portion 14c. Accordingly, when the breaking start portion 96a is broken by the thumb 80 of the unsealing person, the opening portion can be opened in the width direction of the sealing film 92. Therefore, the medical device 12 can be easily taken out to the outside of the container main body 16 through the opening portion opened in the width direction.

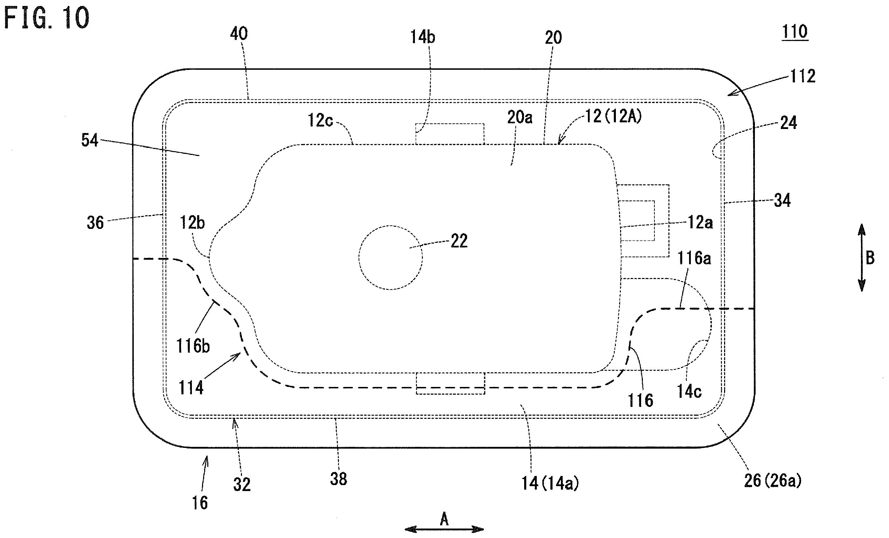

For example, a packaging container 110 according to a second modification example illustrated in FIG. 10 may be employed. In the packaging container 110, a sealing film 112 includes an unsealing assistance portion 114. The unsealing assistance portion 114 has a breaking line 116 along a part of the outer shape of the medical device 12. The breaking line 116 is formed in, for example, a shape formed along one side shape of the medical device 12 in the width direction. The breaking line 116 has a shape formed along the outer shape from the base end 12a to the distal end 12b of the medical device 12.

The breaking line 116 includes a breaking start portion 116a and a breaking extension portion 116b. The breaking start portion 116a is disposed at one end of the breaking line 116 in the longitudinal direction. In a plan view of the packaging container 110 illustrated in FIG. 10, the breaking start portion 116a faces the take-out recess portion 14c of the container main body 16. The breaking extension portion 116b extends from the breaking start portion 116a toward the other end of the breaking line 116 in the longitudinal direction and extends along the outer shape of the medical device 12.

In the packaging container 110, the tip 80a of the thumb 80 is inserted from the breaking start portion 116a of the breaking line 116 into the take-out recess portion 14c of the container main body 16, and thus the sealing film 112 can be broken from the breaking start portion 116a along the breaking extension portion 116b.

Since the sealing film 112 is broken along the breaking line 116 and an opening portion (not illustrated) is formed along the shape of one side portion of the medical device 12 in the width direction, the medical device 12 can be easily and reliably taken out to the outside of the container main body 16 through the opening portion. The position and shape of the breaking line 116 are not particularly limited as long as the breaking line 116 is formed in a shape formed along a part of the outer shape of the medical device 12.

As described above, in a plan view of the packaging container 110 illustrated in FIG. 10, since the breaking line 116 (unsealing assistance portion 114) of the sealing film 112 is formed in a shape along the outer shape of the medical device 12, when the breaking line 116 is broken, the opening portion can be opened along the outer shape of the medical device 12. Therefore, the medical device 12 can be easily taken out to the outside of the container main body 16 through the opening portion.

The above-described embodiment is summarized as follows.

In the above-described embodiment, there is provided a medical device package (10) including a medical device (12) and a packaging container (13, 90, 110) that accommodates the medical device,
in which the packaging container includes
a bottomed container main body (16) including an accommodating recess portion (14b) accommodating the medical device and an opening end portion (24) surrounding the accommodating recess portion, and
a sealing film (18, 92, 112) formed in a sheet shape and bonded to the opening end portion of the container main body to seal the accommodating recess portion,
the container main body includes a take-out recess portion (14c) provided to be adjacent to the medical device and the accommodating recess portion and capable of receiving a finger (80) of an unsealing person, and
the sealing film includes an unsealing assistance portion (56, 94, 114) for facilitating breakage of a part of the sealing film at least at a position facing the take-out recess portion.

The unsealing assistance portion has a breaking line (58, 96, 116) cut out in a thickness direction of the sealing film and extending along the sealing film.

A part of the breaking line faces the medical device.

A plurality of the breaking lines is disposed.

The breaking line is formed of perforations.

When viewed from an opening direction of the opening end portion, the breaking line is formed along a part of the outer shape of the medical device.

The present invention is not limited to the above-described embodiment, and various configurations can be taken without departing from the gist of the present invention.

## Claims

1. A medical device package comprising a medical device and a packaging container that accommodates the medical device, wherein
the packaging container includes
a bottomed container main body including an accommodating recess portion accommodating the medical device and an opening end portion surrounding the accommodating recess portion, and
a sealing film formed in a sheet shape and bonded to the opening end portion of the container main body to seal the accommodating recess portion,
the container main body includes a take-out recess portion provided to be adjacent to the medical device and the accommodating recess portion and capable of receiving a finger of an unsealing person, and
the sealing film includes an unsealing assistance portion for facilitating breakage of a part of the sealing film, at least at a position facing the take-out recess portion.

2. The medical device package according to claim 1, wherein
the unsealing assistance portion has a breaking line cut out in a thickness direction of the sealing film and extending along the sealing film.

3. The medical device package according to claim 2, wherein
a part of the breaking line faces the medical device.

4. The medical device package according to claim 2 or 3, wherein
a plurality of the breaking lines is disposed.

5. The medical device package according to any one of claims 2 to 4, wherein
the breaking line is formed of perforations.

6. The medical device package according to any one of claims 2 to 5, wherein
the breaking line is formed along a part of an outer shape of the medical device when viewed from an opening direction of the opening end portion.
